(19) Europäisches Patentamt
European Patent Office
Office européen des brevets

(11) EP 3 439 621 B1

(12) EUROPEAN PATENT SPECIFICATION

(45) Date of publication and mention of the grant of the patent:
**27.01.2021 Bulletin 2021/04**

(21) Application number: **17707835.9**

(22) Date of filing: **27.02.2017**

(51) Int Cl.:
***A61K 8/02*** *(2006.01)* ***A61K 8/04*** *(2006.01)*
***A61K 8/44*** *(2006.01)* ***A61K 8/73*** *(2006.01)*
***A61K 8/9789*** *(2017.01)* ***A61Q 5/02*** *(2006.01)*
***A61K 8/49*** *(2006.01)* ***A61Q 19/10*** *(2006.01)*

(86) International application number:
**PCT/EP2017/054521**

(87) International publication number:
**WO 2017/174260 (12.10.2017 Gazette 2017/41)**

(54) **AQUEOUS FOAMING CLEANSING COMPOSITIONS COMPRISING N-ACYL SALTS OF POLYCARBOXLIC ACIDS AND PLANT MICROFIBERS**

WÄSSRIGE SCHÄUMENDE REINIGUNGSZUSAMMENSETZUNGEN ENTHALTEND N-ACYL SALZE VON POLYCARBONSÄUREN UND PFLANZLICHE MIKROFASERN

COMPOSITIONS DE NETTOYAGE AQUEUSES MOUSSANTES COMPRENANT DES SELS DES ACIDES N-ACYL POLYCARBOXYLIQUES ET DES MICROFIBRES D'ORIGINE VÉGÉTALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.04.2016 EP 16164298**

(43) Date of publication of application:
**13.02.2019 Bulletin 2019/07**

(73) Proprietors:
- **Unilever PLC**
  **London, Greater London EC4Y 0DY (GB)**
  Designated Contracting States:
  **CY GB IE MT**
- **Unilever N.V.**
  **3013 AL Rotterdam (NL)**
  Designated Contracting States:
  **AL AT BE BG CH CZ DE DK EE ES FI FR GR HR HU IS IT LI LT LU LV MC MK NL NO PL PT RO RS SE SI SK SM TR**

(72) Inventors:
- **VELIKOV, Krassimir, Petkov**
  **3133 AT Vlaardingen (NL)**
- **FLENDRIG, Leonardus, Marcus**
  **3133 AT Vlaardingen (NL)**
- **SHILOACH, Anat**
  **Trumbull, CT 06611 (US)**

(74) Representative: **Fijnvandraat, Arnoldus et al**
**Unilever Patent Group**
**Bronland 14**
**6708 WH Wageningen (NL)**

(56) References cited:
**EP-A1- 2 196 186**
**EP-A2- 2 603 196**
**US-A1- 2003 024 556**
**US-A1- 2014 031 305**
**US-A1- 2014 378 362**

- **KATIE BIRD: "Fiberstar to launch emulsifier alternative for personal care at in-cosmetics", INTERNET CITATION, 16 March 2010 (2010-03-16), page 1, XP002669696, Retrieved from the Internet: URL:http://www.cosmeticsdesign.com/content /view/print/282125 [retrieved on 2012-02-14]**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 439 621 B1

## Description

### Field of the invention

The invention relates to cleansing compositions as defined in the appended claims. The disclosure also relates to N-acyl salts of polycarboxylic acids, especially acylated dicarboxylic acids, (e.g. glutamate, aspartate, and/or malonate surfactants) for use as primary surfactant or primary anionic surfactant (at level greater than any other single surfactant) in aqueous cleansing liquids. Although mild, such surfactants can sometimes be difficult to stabilize; for example, they may not self-structure as well as other anionic surfactants. They also typically provide poor foam stability.

### Background of the invention

N-acyl polycarboxylic acid surfactants (e.g., glutamate or aspartate surfactants) can be desirable for use in liquid cleansers because they are mild to the skin.

However, particularly when used as primary surfactant (or primary anionic surfactant), at least some of these surfactants are difficult to stabilize when used in desirable liquid compositions having pH 6.5 and below. For example, at lower pH, the glutamate surfactants do not readily solubilize. When not fully solubilized, these surfactants are not able to form good micellar phases and, as a consequence, cannot enable the self-structuring of the surfactant based product. In addition, the presence of insoluble particulates from the surfactant may lead to sedimentation and to phase separation. Such products are not commercially viable. The large carboxylic acid head groups of these polycarboxylic acid surfactants also discourages close packing; this encourages head group interactions with metal ions and can cause formation of insoluble soaps. Again, this may lead to sedimentation and phase separation. Poorly soluble surfactants also do not typically foam well.

In short, when used as primary surfactant in lower pH, milder liquid cleanser, N-acyl polycarboxylic surfactants may suffer a number of deficiencies. Less soluble surfactants may be unstable and large carboxylic acid groups can promote formation of insoluble soaps. Further, these surfactants typically form the type of micelles (more spherical than flat or rod-like) which do not have the texture which many consumers desire.

Applicants have now found that, if the surfactants are used as primary surfactant or primary anionic, and are used in combination with defined primary cell wall material, surprisingly it is possible to obtain compositions where structuring is improved; the compositions formed demonstrate improved, superior abilities (e.g., compositions have higher yield stress) and, quite surprisingly (in view of general insolubility of large headgroup surfactant), there is improved foam stability. The cell wall material permits formulation of more viscous compositions which can be squeezed, have good texture (notwithstanding large head groups) and are stable.

Applicants have filed various applications relating to use of glutamate (a polycarboxylic amino acid based surfactant) as primary surfactants. There is no recognition that specific cell wall materials of our invention (e.g., cellulose microfibrils) can be used with the specific polycarboxylic surfactants of our invention to improve viscosity, stability, feel and foam stability. For example, International Application No. PCT/EP2015/076478 to Unilever discloses glutamate as primary surfactant, but there is no teaching in combination with cell wall materials of our invention.

Applicants have also disclosed cleansing material containing primary cell wall materials. PCT/EP2015/081008 for example, discloses cleanser compositions generally, and which contain primary cell wall material. There is no teaching or suggestion anywhere that, when combined with specific types of surfactant (e.g., having large carboxylic acid head groups), there is a synergistic improvement which allows the defined cell wall materials to overcome many of the deficiencies that might be found in a compositions not containing these materials (e.g., deficiencies relating to lower viscosity, texture (feel) and foam formation).

U.S. Publication No. 2015/0159120 to Fernandez-Prieto et al. discloses liquid compositions containing microfibrillated cellulose derived from vegetables or wood, but again there is no disclosure of the specific surfactant of our invention or how to overcome problems associated with these specific surfactants. EP 2 603 196 discloses shampoo compositions with mild surfactants and citrus fibers.

### Summary of the invention

Specifically, the invention comprises personal care cleansing compositions (liquid or gel-like cleansing compositions) which contain:

1) 0.5 to 35% by wt. of total composition of a surfactant system which contains:

a) 0.5 to 25% by wt. of total composition anionic surfactant and
b) 0 to 20% by wt. of a co-surfactant selected from the group consisting of non-ionic surfactant, cationic surfactant,

amphoteric surfactant and mixtures thereof; where an N-acyl salt of polycarboxylic acid, an anionic surfactant, is present at a level of 50% or more of all surfactant (both (a) and (b)); or is present at a level of 50% or more of anionic surfactant (a);

2) 0 to 30% of a hair or skin benefit agent;
3) 0.05 to 4.0% of a primary cell wall material comprising cellulose microfibrils, wherein:

a) the primary cell wall material is sourced from plant parenchymal tissue;
b) 80% or more, preferably 80% to 100%, more preferably 85% to 100% of the microfibrils have a diameter of less than 50 nanometers (nm); and

4) Balance of water,

wherein the primary cell wall material is defibrillated,
wherein the cleansing composition has a composition homogeneity parameter (CHP) of 0.030 and greater, according to the protocol as defined herein.

The liquid cleansing composition has a composition homogeneity parameter (CHP) of 0.030 and greater.

The primary cell wall material is defibrillated. The cell wall material has a fiber homogeneity parameter (FHP) of 0.030 and greater.

Preferably, the cell wall has a fiber defibrillation parameter (FDP) of 0.10 Hz or greater.

The cleansing compositions of the present invention are typically in a liquid, gel or paste format.

Cleansing compositions featuring desirable properties including enhanced foam stability can suitably be prepared by methods including a high shear treatment step. Disclosed is also a method for preparing a cleansing composition, wherein the cleansing composition comprises

a. water;
b. 0.5 to 35 by wt % of surfactant system containing 0.5 to 25 % by wt. anionic surfactant and 0 to 20% co-surfactant as defined above; and
c. 0.05 to 4.0 wt % primary cell wall material (preferably defibrillated) comprising cellulose microfibrils; and wherein

- the primary cell wall material is sourced from plant parenchymal tissue,
- at least 80 wt % of the microfibrils are smaller than 50 nm in diameter; and wherein the method comprises the steps of

i. providing a source of primary cell wall material;
ii. dispersing the primary cell wall material in an aqueous phase, thereby to form an aqueous dispersion comprising between 0.05 to 4.0 wt. % of the primary cell wall material;
iii. treating the aqueous dispersion to obtain a dispersion comprising the primary cell wall material, whereby the treatment includes a high shear treatment step selected from high pressure homogenisation at a pressure of between 500 and 2000 bar or microfluidising at a pressure of between 500 and 2000 bar;

wherein other constituents of the cleansing composition are independently mixed into the aqueous phase before step ii, between steps ii and iii, or after step iii.

Disclosed is also a method for preparing a cleansing composition, wherein the cleansing composition comprises

a. water;
b. 0.5 to 35 by wt. % of surfactant system as defined above; and
c. 0.05 to 4.0 wt. % primary cell wall material (preferably defibrillated) comprising cellulose microfibrils;

and wherein

- the primary cell wall material is sourced from plant parenchymal tissue,
- at least 80 wt % of the microfibrils are smaller than 50 nm in diameter;

and wherein the method comprises the steps of

i. providing a source of primary cell wall material;

ii. dispersing the primary cell wall material in an aqueous phase, thereby to form an aqueous dispersion comprising between 0.05 to 4.0 % by wt. of the primary cell wall material; and

iii. treating the aqueous dispersion to obtain a dispersion comprising primary cell wall material, whereby the treatment includes one or more high shear treatment steps and wherein the treatment is such that the fibre defibrillation parameter FDP of the primary cell wall material is at least 0.10 Hz or the fibre homogeneity parameter FHP of the primary cell wall material is at least 0.022;

wherein other constituents of the cleansing composition are independently mixed into the aqueous phase before step ii, between steps ii and iii, or after step iii.

The methods yield cleansing compositions displaying desirable properties, including the aforementioned enhanced foam stability. Therefore, the invention also provides a cleansing composition obtainable by either of the two methods noted above.

Preferably, the invention provides use of defibrillated cell wall material comprising microfibrils to increase the foam stability of a cleansing composition comprising water and 0.5 to 35% by wt. of defined surfactant system, wherein the composition has a composition homogeneity parameter CHP of at least 0.030.

Preferably, the invention provides use of defibrillated cell wall material comprising microfibrils to increase the foam stability of a cleansing composition comprising water and 0.5 to 35% by wt. of desired surfactant system, wherein the composition has a fibre defibrillation parameter FDP of at least 0.010 Hz.

## Detailed description of the invention

The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Unless specified otherwise, numerical ranges expressed in the format "from x to y" are understood to include x and y. When for a specific feature multiple preferred ranges are described in the format "from x to y", it is understood that all ranges combining the different endpoints are also contemplated. For the purpose of the invention ambient temperature is defined as a temperature of about 20 degrees Celsius.

Cleansing composition

The cleansing composition is a composition intended for personal care cleansing. The cleansing composition preferably is in a liquid, gel or paste format, more preferably it is in a liquid format. Thus, it is preferred that the cleansing composition according to the present invention is a liquid cleansing composition. The precise format and formulation of the composition can suitably be adapted to the intended type of application, as is generally known by the skilled person. For example, a preferred format would be a wash-off personal wash cleanser composition. However, other types of cleansing compositions are also contemplated. The cleansing composition comprises water, surfactant system as defined, and primary cell wall material, preferably defibrillated. In addition, the cleansing composition may suitably comprise other ingredients that are typical for such personal wash cleanser cleansing compositions. For example, the composition may also comprise skin benefit agents, structurants, preservatives, etc.

Anionic Surfactant

A key to the invention is that there is present 0.5 to 35% by wt. of total composition of a surfactant system wherein anionic surfactant comprises 0.5 to 25% by wt. of total composition and wherein N-acyl salt of polycarboxylic acid, preferably salt of acylated dicarboxylic acid (e.g., glutamate, aspartate, malonate) comprises 50% or more of all surfactant ("primary surfactant"); or 50% or more, preferably 60% or more by wt. of the total anionic surfactant present (even if anionic overall is less than 50% of total surfactant), and is present in an amount equal to or greater than any other single surfactant in the composition. Preferably anionic surfactant comprises 1 to 15% by wt. of total composition, more preferably 2 to 12% of total composition. In some compositions, anionic comprises 5 to 12% by wt. of the total composition and surfactants which are not anionic comprise 1 to 7% by wt. of the composition. The amount of N-acyl salt of polycarboxylic acid should be maximized and as noted, even if other surfactants are present in amounts greater than anionic (e.g., when salt of polycarboxylic acid is not the "primary surfactant"), the surfactant is present at 50% or more of anionic surfactant and is present in an amount equal to or greater than any other single surfactant present.

The salt may be a salt of polycarboxylic acid such as glutamate or malonate. Preferred polycarboxylic surfactants include dicarboxylic surfactants such as glutamates or apartates and mixtures thereof.

For example, the salt may be a salt of acyl glutamate. Such salt has a structure as follows:

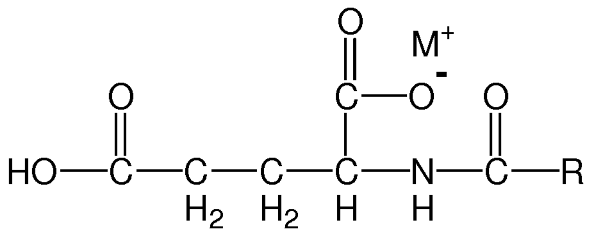

or

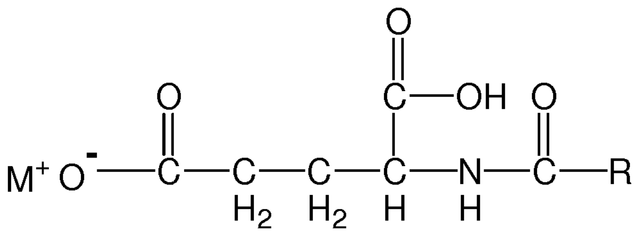

(It is noted that one or the other structures will occur at pH levels of the invention (pH 6.5 and below, preferably 3 to 6.5) and that at higher pH (e.g., 8 or 9), some di-salt is also present),
where R is alkyl or alkenyl group (generally saturated although some unsaturated, for example, oleoyl, may be present) having 8 to 20 carbons, preferably 8 to 16 carbons, more preferably 10 to 14 carbons. Preferably, R is predominantly a mixture of $C_{10}$ to $C_{14}$.

M is a solubilizing cation such as, for example, sodium, potassium, ammonium or substituted ammonium. It is preferred that the cation is sodium or potassium, more preferably sodium. Sodium salt is preferred.

Amino acid surfactants are typically commercially available in various formats. One is powder or flake and the other is liquid.

Examples of solid dicarboxylic amino acid surfactants include:

- sodium N-cocoyl-L -glutamate (e.g., Amisoft® CS-11 by Ajinomoto)
- sodium N-lauroyl-L- glutamate (e.g., Amisoft® LS-11 by Ajinomoto)
- sodium N-myristoyl-L-glutamate (Amisoft® MS-11 by Ajinomoto)
- potassium N-cocoacyl_l-Glutamate (e.g., Amisoft® CK-11 by Ajinomoto)
- potassium N-myristoyl-L-glutamate (Amisoft® MK-11 by Ajinomoto)
- potassium N-lauroyl-L-glutamate (Amisoft® LK-11 by Ajinomoto)
- Sodium Lauroyl Aspartate (AminoFoamer™ FLMS-P1 by Asahi Kasei Chemical Corporation)
- Sodium Lauroyl Glutamate (Aminosurfact™ ALMS-P1/S1 by Asahi Kasei Chemical Corporation)
- Sodium Myristoyl Glutamate (Aminosurfact™ AMMS-P1/S1 by Asahi Kasei Chemical Corporation)

Liquid amino acid surfactants typically contains 20-35% surfactant active, high in pH and inorganic salt (e.g. from 3 to 6% NaCl). Examples include:

- AMISOFT® ECS-22SB: Disodium Cocoyl Glutamate (30% Aqueous Solution)
- AMISOFT® CS-22: Disodium Cocoyl Glutamate sodium Cocoyl Glutamate (25% Aqueous Solution)
- AMISOFT® CK-22: Potassium Cocoyl Glutamate (30% Aqueous Solution)
- AMISOFT® LT-12: TEA-Lauroyl Glutamate (30% Aqueous Solution)
- AMISOFT® CT-12 TEA-Cocoyl Glutamate (30% Aqueous Solution)
- Aminosurfact™ ACDS-L: Sodium Cocoyl Glutamate (25% Aqueous Solution)
- Aminosurfact™ ACDP-L: Potassium Cocoyl Glutamate(22%)+Sodium Cocoyl Glutamate(7%)
- Aminosurfact™ ACMT-L: TEA-Cocoyl Glutamate(30% Aqueous Solution)
- AminoFoamer™ FLDS-L: Sodium Lauroyl Aspartate (25% Aqueous Solution)

The pH of the overall composition is typically 6.5 and lower, preferably 6.0 and lower. Preferably pH is 3 to 6.5 and more preferably 3 to 6. More preferably, pH is 3.5 to less than 6, preferably 3.5 to 5.5, more preferably 4.0 to 5.5, even more preferably 4.0 to 5.1.

While the salt of polycarboxylic acid (e.g., acyl glutamate, acyl aspartate) may be used as the only anionic

surfactant in the total composition, it is desirable to use other anionic surfactants, subject to the levels defined here. One preferred co-anionic (as opposed to co-surfactant 1(b)) is sarcosinate (alkyl salt of $C_{10}$-$C_{14}$ acyl sarcosinate is a preferred sarcosinate, where salt is defined as in M above). Another preferred co-anionic is a taurate. A salt of $C_{10}$-$C_{14}$ acyl taurates (e.g., sodium cocoyl methyl taurates) is preferred. Generally, it is preferred not to use salts which would tend to precipitate at lower pH values. Thus, it is preferred to minimize, for example, the amount of acyl glycinate (<1.0%, preferably <0.5%, preferably absent altogether).

Generally, sarcosinates have formula:

$$R^2CON(CH_3)CH_2CO_2M;$$

Taurates have formula:

$$R^2CONR^3CH_2CH_2SO_3M,$$

where $R^3$ is methyl;

Glycinates have formula:

$$R^2CONHCH_2CO_2M$$

where $R^2$ above is alkyl or alkenyl having 8 to 22 carbons, preferably 12 to 18 carbons; and

M is solubilizing cation as defined above.

Compositions of the invention may have low levels of alkyl ether sulfates, for example, sodium lauryl ether sulfate. By low is meant less than 20% of anionic, preferably less than 10%, more preferably less than 5%. In some embodiments the compositions have less than 0.5% alkyl ether sulfate and in some there is substantially no alkyl ether sulfate. These types of sulfates are preferably minimized because they are less mild than other surfactants.

Co-Surfactant

A second component of the invention may comprise 0% to 20%, preferably 0.5 to 15% by wt. of total composition of a co-surfactant selected from the group consisting of non-ionic, cationic, and amphoteric surfactant and mixtures thereof.

Preferred co-surfactants are amphoteric or zwitterionic surfactant. Preferably, the co-surfactant is amphoteric.

This general class of amphoteric detergents has the following general structure:

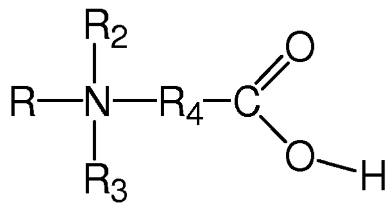

where R is an alkyl or alkenyl radical of 7 to 17 carbons or a carboxamido functional group of the general structure

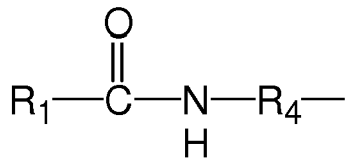

where $R_1$ is an alkyl or alkenyl radical of 7 to 17 carbons and $R_4$ is an alkyl, hydroxyalkyl, or carboxyalkyl radical of 1 to 3 carbons. $R_2$ and $R_3$ are each independently a proton, an alkyl, hydroxyalkyl, or carboxyalkyl radical of 1 to 3 carbons, or is missing entirely, subject to the following restraints. When $R_2$ and $R_3$ are each independently an alkyl, hydroxyalkyl,

or carboxyalkyl radical, the nitrogen in a quaternary amine and is a cationic charge center. When one of $R_2$ or $R_3$ is an alkyl, hydroxyalkyl, or carboxyalkyl radical and the other is a proton or is missing entirely, the nitrogen is a tertiary amine. At a pH well below the $pK_a$ of the tertiary amine, the other of $R_2$ or $R_3$ will be a proton and the amine will be a cationic charge center. At a pH well above the $pK_a$ of the tertiary amine, the other of $R_2$ or $R_3$ will be missing entirely and the amine will be a neutral charge center.

Preferred examples of amphoteric noted above include cocoamidopropylbetaine (CAPB), $C_{10}$-$C_{14}$ alkyl betaine, the salt of $C_{10}$-$C_{14}$ alkyl amphoacetate (e.g. lauroamphoacetate) and mixtures thereof.

Another class of amphoteric detergents are the sultaines having the following general structure:

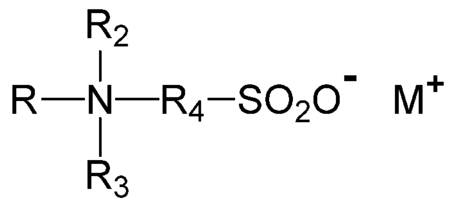

where R is an alkyl or alkenyl radical of 7 to 17 carbons or a carboxamido functional group of the general structure

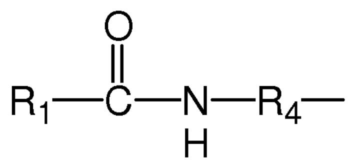

where $R_1$ is an alkyl or alkenyl radical of 7 to 17 carbons and $R_4$ is an alkyl, hydroxyalkyl, or carboxyalkyl radical of 1 to 3 carbons. $R_2$ and $R_3$ are each independently an alkyl, hydroxyalkyl, or carboxyalkyl radical of 1 to 3 carbons, so that the nitrogen in a quaternary amine and is a cationic charge center. A preferred amphoteric surfactant of this class is cocamidopropyl hydroxy sultaine (CAPHS), lauramidopropyl hydroxy sultaine (LAPHS), or lauryl hydroxy sultaine (LHS).

Primary cell wall material

For the purpose of the invention "primary cell wall material" is defined as the cell wall material from which essentially all cold water soluble components have been removed, i.e. at a temperature of around 20 degrees Celsius. This can easily be achieved by washing with water.

The primary cell wall material is sourced (i.e. prepared) from plant parenchymal tissue. The microfibrils in the cleansing composition according to the invention are microfibrils obtained from primary cell wall material. The source of the plant parenchyma cells may be any plant that contains plant parenchyma cells having a cellulose skeleton. A plant cell wall typically contains cellulose and hemicellulose, pectin and in many cases lignin. This contrasts with the cell walls of fungi (which are made of chitin), and of bacteria, which are made of peptidoglycan. Primary plant cell walls contain lignin only in minor amounts, if at all. The primary cell wall material used in the cleansing composition according to the invention may comprise some lignin, like less than 10 wt % calculated on total amount of cell wall material, but preferably does not contain substantial amounts of lignified tissue. Preferably the primary cell wall material consists essentially of non-lignified tissue as understood by the skilled person in the area of plant biology.

Preferably, the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit.

The primary cell wall material may optionally have undergone several pre-treatment steps before it is brought in the defibrillated state. Such pre-treatments include but are not limited to heating, cooking, washing, refining, depectinating, as long as the defibrillated cell wall material comprising microfibrils is present in the cleansing composition as required by the present invention. Hence, the parenchymal tissue may for instance also be provided in the form of a puree.

Microfibrils

In the context of the present invention, the microfibrils present in or derived from the primary cell wall material, are the strongly self-associated fibrous structures typically found in plant cell walls. In the native plant tissue, they are

conventionally present in the form of aggregates from a few tens of nanometres to a few micrometres. These aggregates consist of the elementary microfibrils. These elementary microfibrils are well-known. A typical microfibril generally comprises about 36 aligned beta-1-4-glucose polymer chains.

The cleansing composition according to the invention comprises 0.05 to 4.0% by wt. of primary cell wall material (which is defibrillated) comprising microfibrils. Here, the wt % of the total composition is based on the dry weight of the primary cell wall material from which essentially all cold water soluble components have been removed (i.e. the insoluble fraction, which is also understood as the fibre fraction). The amount of cell wall material may suitably be selected to obtain the desired effect and depends on the overall product format. It may for instance also depend on the typical level of dilution upon application and the amount of cell wall material required in the lather upon its formation to provide the enhanced foam stability to the lather. Preferably, the amount of cell wall material in the cleansing composition according to the invention is from 0.2 to 2.5 wt %, more preferably from 0.3 to 2 wt %, more preferably from 0.5 to 1.5 wt % and even more preferably from 0.7 to 1.2 wt %.

Preferably, the microfibrils are obtained from the primary cell wall material by removing soluble and unbound sugars, protein, polysaccharides, oil soluble oils, waxes and phytochemicals (e.g. carotenoids, lycopene). This is suitably achieved using well known techniques including cutting up the cell wall material, cooking, washing, centrifugation, decanting and drying as is well-known to the skilled person.

Preferably the primary cell wall material comprises at least 50 wt % of microfibrils, more preferably at least 60 wt %, even more preferably at least 70 wt %, still more preferably at least 80 wt %, even still more preferably at least 90 wt % and most preferably the primary cell wall material consists essentially of microfibrils. Here, the wt % is based on the dry weight of the primary cell wall material and the microfibrils.

Plant cell walls, especially in parenchymal tissue contain hemicelluloses and pectin in addition to cellulose. Thus, the microfibrils in the primary cell wall material may typically comprise cellulose, hemicellulose, and pectin. However, the primary cell wall material of the invention does not necessarily contain hemicellulose and/or pectin. The hemicellulose or part thereof may have been removed when the primary cell wall material is prepared from the plant parenchymal tissue. Therefore, the primary cell wall material of the invention optionally comprises hemicellulose, like for example in an amount of 0 to 40 wt %. Preferably the primary cell wall material comprises hemicelluloses, preferably in an amount of up to 40 wt %, like for example from 5 to 40 wt %, and more preferably in an amount from 10 to 30 wt %.

Likewise the pectin or part thereof may have been removed when the primary cell wall material is prepared from the plant parenchymal tissue. Therefore, the primary cell wall material of the invention optionally comprises pectin, like for example in an amount of 0 to 30 wt %. Preferably the primary cell wall material comprises pectin, preferably in an amount of up to 30 wt %, like for example from 5 to 30 wt %, and more preferably in an amount from 10 to 20 wt %.

Preferably, the primary cell wall material of the invention comprises hemicelluloses and pectin.

The primary cell wall material in the cleansing composition of the invention preferably comprises defibrillated cell wall material, i.e. the microfibrils that make up the fibers present in the primary cell wall are at least partially disentangled without breaking them. The degree of disentanglement helps provide the cleansing composition of the invention with its surprising properties. The CHP, FHP and FDP parameters all correlate to this degree of disentanglement.

Preferably, the average length of the microfibrils from the defibrillated primary cell wall material is more than 1 micrometer and preferably more than 5 micrometers.

At least 80 wt % of the microfibrils is smaller than 50 nm in diameter. Preferably at least 80 wt % of the microfibrils is smaller than 45 nm in diameter, more preferably smaller than 35 nm, even more preferably smaller than 20 nm and still more preferably smaller than 10 nm. The microfibril diameter can be suitably determined using the method described in the Examples section below.

The primary cell wall material may be suitably defibrillated by subjecting it to mechanical energy and/or cavitation thereby disentangling the cellulose-containing microfibrils. This can be done as part of the process for obtaining the microfibrils from the primary cell wall material, thus resulting in isolated defibrillated cell wall material comprising microfibrils. Alternatively, the primary cell wall material can be combined with one or more of the other ingredients of the cleansing composition (including for example the surfactant) wherein the resulting mixture is subjected to mechanical energy and/or cavitation thereby disentangling the microfibrils in the plant fiber source. The required level defibrillation can also be arrived at by a succession of various such disentanglement treatments, for example by first subjecting a dispersion of the primary cell wall material to a high shear treatment, and at later stage subjecting a premix of the cleansing composition to another high shear treatment. Alternatively, if the pre-processing of the primary cell wall material provides sufficient disentanglement to yield the required level of defibrillation in the final cleansing composition, it may suffice if the manufacturing steps in which the primary cell wall material is combined with the other constituents of the cleansing composition include only mixing steps of relatively low shear.

The cellulose in the microfibrils in the defibrillated primary cell wall material in any of the compositions of the present invention preferably has an average degree of crystallinity of less than 50%. Preferably the average degree of crystallinity of the cellulose in the microfibrils is less than 40%, more preferably less than 35% and even more preferably less than 30%. The table below shows the average degree of crystallinity of typical sources of cellulose microfibrils. It

shows that the cellulose in primary cell wall material sourced from plant parenchymal tissue typically has a degree of crystallinity of less than 50 wt %.

**Table 1:** Average degree of crystallinity of cellulose (all polymorph cellulose I)

| **Source** | **Average degree of crystallinity (%)** |
|---|---|
| Tomato fibers | 32 |
| Citrus fiber (Citrus Fiber AQ+N) | 29 |
| Nata de Coco | 74 |
| Cotton | 72 |
| Wood pulp fiber (Meadwestvaco) | 61 |
| Sugar beet fibre (Nordix Fibrex) | 21 |
| Pea fibres (PF200vitacel) | 42 |
| Oat fibres (780 Sunopta) | 43 |
| Corn hull (Z-trim) | 48 |
| Sugar cane Fiber (Ultracel) | 49 |

The average degree of crystallinity can be suitably determined according to the described in the Examples section below.

*The composition homogeneity parameter CHP*

The cleansing composition of the invention has a composition homogeneity parameter (CHP) of at least 0.030. The CHP provides a measure for the extent to which the primary cell wall material has been defibrillated, based on confocal scanning laser microscopy (CSLM) performed on a standardised sample comprising the defibrillated cell wall material. The CHP of the cleansing composition is established by the following protocol. The protocol to establish the parameter includes three parts: sample preparation, CSLM microscopy to obtain micrographs of the sample, and digital image analysis to calculate the CHP value.

Thus, the protocol includes the sample preparation steps of

a. preparing 150 ml of an aqueous, concentration-standardised sample at room temperature from the cleansing composition, wherein the concentration-standardised sample comprises the microfibrils contained in the defibrillated primary cell wall material at a concentration of 0.25 wt % with respect to the weight of the standardised sample;
b. evenly distributing the primary cell wall material over the concentration-standardised sample volume by agitating the sample with a Silverson overhead mixer equipped with a small screen having 1 mm holes at 2000 rpm for 60 seconds;
c. dying the microfibrils by providing a 0.5 wt. % $_{[VK5]}$aqueous stock solution of Congo Red dye and contacting an aliquot of the standardised sample with an amount of the Congo Red solution, wherein the amount is 1.0 vol-% with respect to the volume of the aliquot of the standardised sample;
d. filling a sample holder suitable for performing CSLM with an aliquot of the dyed standardised sample.

In step c, for example, 2 mL of the standardised sample is contacted with 20 μl of the Congo Red solution. In order to ensure even distribution of the dye throughout the sample, it may for instance be gently shaken.

The sample holder of step d suitably includes two cover slides separated by a spacer comprising a bore of sufficient volume to enable the recording of sufficient micrographs for digital image analysis as described below.

To obtain micrographs, the protocol includes the following step:

e. imaging the dyed standardised sample with a confocal scanning laser microscope equipped with a diode-pumped solid state laser emitting at a wavelength of 561 nm and operated at a fixed laser power, using a 10x objective with a numerical aperture of 0.40, and thereby recording at least 25 independent micrographs at a resolution of 1024x1024 pixels where each pixel represents a sample size of within the range of 1490 by 1490 nm to 1540 by 1540 nm, adjusting the intensity and gain settings such that in every image between 0.1 and 5% of the pixels are saturated and recording the micrographs at a colour depth of at least 8 bits per pixel.

The CHP is a measure relating to the primary cell wall material. Therefore, micrographs should be recorded

whilst avoiding imaging of air bubbles or the sample edge. Likewise, care should be taken to avoid imaging other objects of macroscopic dimensions that do not originate from the defibrillated primary cell wall material. This may conveniently be accomplished for instance by removing such objects of macroscopic dimensions during sample preparation in step a or by avoiding them in the sample whilst collecting micrographs.

Typically, one or more photomultiplier tubes are used as the light detectors in the microscope. Preferably the microscope is equipped with three photomultiplier tubes (PMTs). Independent micrographs are micrographs that are non-overlapping, both in the x-y plane and in the z-direction. The micrographs may suitably be recorded at a colour depth higher than 8 bits (for instance at 24 bit RGB), since this can easily be converted to a lower colour depth by well-known means.

The digital image analysis part of the protocol involves the following steps:

f. ensuring that the micrographs are present as or converted to a format with a single intensity value for each pixel;
g. normalising each individual micrograph by recalculating the pixel values of the image so that the range of pixel values used in the image is equal to the maximum range for the given colour depth, thereby requiring 0.4% of the pixels to become saturated;
h. obtaining for each individual micrograph the image histogram and removing spikes from each histogram by visual inspection;
i. for each individual image histogram determining the full width at half maximum (FWHM), by first determining the maximum count in the histogram and the channel containing this maximum count (the maximum channel), then counting the number N of channels between the first channel containing a value equal or higher than half the maximum and the last channel containing a value equal or higher than half the maximum thereby including this first and last channel in the count N, and then calculating the FWHM by dividing the count N by the total number of channels;
j. calculating the composition homogeneity parameter CHP, wherein CHP is the average of the FWHM values obtained for the individual micrographs.

The digital image analysis steps may suitably be carried out using well-known image analysis software including for instance ImageJ. The result of step f should be that the image is of a format wherein the intensity for each pixel is expressed as a single value.

This is for instance the case if the image is a "grey-scale" image. In contrast, images in RGB format or a related format having three intensity values per pixel should be converted. This is easily achieved by well-known operations in the field of digital image analysis. An example of a suitable output format would be a grey-scale image with 8bits per pixel.

The normalising operation of step g is generally known as a histogram stretch operation or a contrast stretch operation. The normalisation is performed by allowing a small percentage of pixels in the image to become saturated. Here saturation includes both the minimum and maximum value for the given colour depth. In an 8 bit greyscale image, the minimum value would be 0 and typically displayed as black, whilst the maximum value would be 255 and typically displayed as white. The image histogram of step h is a well-known property for digital images, representing the distribution of the pixels over the possible intensities, by providing the pixel count for each intensity channel. For the purpose of the spike-removal of step h, the value for a particular channel is considered a spike if it is considerably higher than the values of the adjacent channels, typically at least a factor of 1.5 higher. The lower half-maximum channel in step i corresponds to the channel containing a count of half the maximum count that is furthest away from the maximum channel on the low-intensity side of the maximum channel. Analogously, the upper half-maximum channel corresponds to the channel containing a count of half the maximum count that is furthest away from the maximum channel on the high-intensity side of the maximum channel. The FWHM that is obtained in step i will be a value between 0 and 1.

A preferred way of establishing the CHP for the cleansing composition is by following the protocol in the way described in the Examples section below. The above protocol and the Examples provide methods of measuring the CHP. However, the CHP may also be determined by a different protocol, as long as that protocol would lead to the same physical result, i.e. it would yield the same CHP for a particular cleansing composition as the above protocol.

The cleansing composition preferably has a composition homogeneity parameter CHP of at least 0.031, more preferably at least 0.032, even more preferably at least 0.033, even more preferably at least 0.040 and still more preferably at least 0.050. Preferably, the cleansing composition has a CHP of at most 0.20, more preferably at most 0.15, and even more preferably at most 0.10.

The fibre homogeneity parameter FHP

Preferably, the degree of defibrillation of the primary cell wall material in the cleansing composition is suitably characterised by the fibre homogeneity parameter FHP. Like the CHP, the FHP is measured based on analysis of CSLM micrographs, but differs in the way the sample is prepared. The FHP is defined for the defibrillated primary cell wall material dispersed in water. That is, the FHP is determined for the separate (defibrillated) primary cell wall material, not

for the formulated cleansing composition.

Thus, the defibrillated primary cell wall material of the cleansing composition according to the fourth aspect of the invention has a fibre homogeneity parameter FHP of at least 0.022. The defibrillated primary cell wall material preferably has a fibre homogeneity parameter FHP of at least 0.025, more preferably at least 0.030, even more preferably at least 0.035, still more preferably at least 0.040, yet more preferably at least 0.045 and still more preferably at least 0.050. The defibrillated primary cell wall material preferably has a fibre defibrillation parameter FHP of at most 0.20, more preferably at most 0.15 and even more preferably at most 0.10.

The protocol to establish the FHP includes three parts: sample preparation, CSLM microscopy to obtain micrographs of the sample, and digital image analysis to calculate the FHP value, analogous to the protocol to establish the CHP.

Thus, the protocol includes the sample preparation steps of

a) preparing 300 ml of a concentration-standardised sample at room temperature of the defibrillated primary cell wall material, wherein the concentration-standardised sample comprises the microfibrils contained in the defibrillated primary cell wall material at a concentration of 0.100 wt % with respect to the weight of the standardised sample;
b) evenly distributing the primary cell wall material over the concentration-standardised sample volume by agitating the sample with a Silverson overhead mixer equipped with a small screen having 1 mm holes at 2000 rpm for 60 seconds;
c) dying the microfibrils by providing a 0.5 %-w/v aqueous stock solution of Congo Red dye and contacting an aliquot of the standardised sample with an amount of the Congo Red solution, wherein the amount is 1.0 vol-% with respect to the volume of the aliquot of the standardised sample;
d) filling a sample holder suitable for performing CSLM with an aliquot of the dyed standardised sample.

The standardised sample of the defibrillated primary cell wall material may be prepared in different ways, which may be appropriately selected depending on the preparation conditions of the defibrillated primary cell wall material and/or the cleansing composition. Thus for example, the standardised sample may suitably be prepared by using a dispersion consisting essentially of the defibrillated primary cell wall material dispersed in water, wherein the dispersion results from a defibrillation process. This is particularly useful, if the primary cell wall material is subjected to a defibrillation step before it is contacted with other constituents of the cleansing composition. A possible alternative is to separate the primary cell wall material from the other constituents of the cleansing composition, after the latter has been prepared.

To obtain micrographs, the protocol includes the following step:

e) imaging the dyed standardised sample with a confocal scanning laser microscope equipped with a diode-pumped solid state laser emitting at a wavelength of 561 nm and operated at a fixed laser power, using an oil-immersed 40x objective with a numerical aperture of 1.25, and thereby recording at least 25 independent micrographs at a resolution of 1024x1024 pixels where each pixel represents a sample size of within the range of 350 by 350 to 400 by 400nm, adjusting the intensity and gain settings such that in every image between 0.1 and 5% of the pixels are saturated and recording the micrographs at a colour depth of at least 8 bits per pixel.

Notably, the objective lens (i.e. an oil-immersed 40x objective) used in the protocol to determine the FHP differs from that used in the protocol to determine the CHP (i.e. a 10x objective).

The further parts of the protocol to determine the FHP - namely the digital image analysis - follows the same steps as steps f to j of the protocol described hereinabove for the determination of the CHP, with the proviso that in step j, the fibre homogeneity parameter FHP is calculated as the average of the FWHM values obtained for the individual micrographs.

A preferred way of establishing the FHP for the cleansing composition is by following the protocol in the way described in the Examples section below for the CHP, whilst taking into account the above differences between the methods to measure the CHP and the FHP. The above protocol and the Examples provide methods of measuring the FHP. However, the FHP may also be determined by a different protocol, as long as that protocol would lead to the same physical result, i.e. it would yield the same FHP for a particular cleansing composition as the above protocol.

The fibre defibrillation parameter FDP

Preferably, the degree of defibrillation of the primary cell wall material in the cleansing composition is suitably characterised by the fibre defibrillation parameter FDP. The FDP provides a measure for the extent to which the primary cell wall material has been defibrillated, based on an NMR (nuclear magnetic resonance) method performed on a standardised sample comprising the defibrillated cell wall material. Like the FHP, the FDP is defined for the defibrillated primary cell wall material dispersed in water. That is, the FDP is determined for the separate primary cell wall material, not for the fully formulated cleansing composition.

Thus, the defibrillated primary cell wall material of the cleansing composition according to the third aspect of

the invention has a fibre defibrillation parameter FDP of at least 0.10 Hz. The defibrillated primary cell wall material preferably has a fibre defibrillation parameter FDP of at least 0.11 Hz, more preferably at least 0.12 Hz, even more preferably at least 0.13 Hz, even more preferably at least 0.15 Hz and still more preferably at least 0.18 Hz. The defibrillated primary cell wall material preferably has a fibre defibrillation parameter FDP of at most 0.50 Hz, more preferably at most 0.40 Hz, even more preferably at most 0.30 Hz and still more preferably at most 0.20 Hz.

The protocol to establish the fibre defibrillation parameter FDP includes three parts: sample preparation, NMR measurement to collect CPMG relaxation decay data, and data analysis to calculate the FDP value.

Thus, the protocol includes the sample preparation steps of

a. preparing 300 ml of a concentration-standardised sample at room temperature of the defibrillated primary cell wall material, wherein the concentration-standardised sample comprises the microfibrils contained in the defibrillated primary cell wall material at a concentration of 0.100 wt % with respect to the weight of the standardised sample;
b. evenly distributing the primary cell wall material over the concentration-standardised sample volume by agitating the sample with a Silverson overhead mixer equipped with a small screen having 1 mm holes at 2000 rpm for 60 seconds;
c. adjusting the pH of the concentration-standardised sample to 3.3 ± 0.1;
d. transferring an aliquot of the concentration- and pH-standardised sample to a flat-bottom NMR tube of 10 mm diameter, ensuring a fill height such that upon placement of the sample in the NMR spectrometer of step h, the fill height is within the region where the radiofrequent field of the coil of the NMR spectrometer is homogeneous.

The standardised sample of the defibrillated primary cell wall material may be prepared in different ways, which may be appropriately selected depending on the preparation conditions of the defibrillated primary cell wall material and/or the cleansing composition. Thus, for example, the standardised sample may suitably be prepared by using a dispersion consisting essentially of the defibrillated primary cell wall material dispersed in water, wherein the dispersion results from a defibrillation process. This way of preparing the standardised sample is preferred and is particularly useful if the primary cell wall material is subjected to a defibrillation step before it is contacted with other constituents of the cleansing composition. A possible alternative is to separate the primary cell wall material from the other constituents of the cleansing, after the latter has been prepared.

The distributing step b is intended to provide an even distribution of the microfibril material over the sample volume, whilst having a limited and controlled effect on the level of defibrillation of the sample. In step c, the pH is suitably standardised with the aid of citric acid. The optimal fill height in step d may depend on the type of NMR spectrometer used, as known by the skilled person. It will typically be about 1 cm.

In the further steps of the protocol, the concentration- and pH-standardised sample will be referred to as the standardised sample.

The data analysis requires comparison of a $T_2$ distribution curve (see below) of the standardised sample with a matrix reference sample, which should preferably be essentially free from microfibril material. Therefore, the protocol also includes the step of:

e. preparing a matrix reference sample by centrifuging an aliquot of the standardised sample in a 2 ml Eppendorf cup at a relative centrifugation force of 15000 for 10 minutes and transferring the supernatant to a flat-bottom NMR tube of 10 mm diameter, ensuring a fill height such that upon placement of the sample in the NMR spectrometer of step h, the fill height is within the region where the radiofrequent field of the coil of the NMR spectrometer is homogeneous.

Subsequently, to collect and analyse the data, the protocol includes the steps of:

f. equilibrating the NMR tubes at a temperature of 20 °C;
g. recording relaxation decay data for the standardised sample at 20 °C on an NMR spectrometer operating at a proton resonance frequency of 20 MHz, using a CPMG (Carr Purcell Mayboom Gill) $T_2$ relaxation pulse sequence, with a 180° pulse spacing of 200 microseconds, and a recycle delay time of 30 seconds;
h. recording relaxation decay data for the matrix reference sample under the same conditions as in step h;
i. performing inverse Laplace transformation to the obtained decay data for both the standardised sample and the matrix reference sample, requiring $T_2$ to be in the range of 0.01 to 10 seconds;
j. identifying in the $T_2$ distribution curve of the standardised sample the peak corresponding to the water protons of which the $T_2$ is averaged by exchange between the bulk water phase and the surface of the defibrillated primary cell wall material and identifying in the $T_2$ distribution curve of the matrix reference sample the peak corresponding to the bulk water phase;
k. calculating $T_2$(sample), which is defined as the weighted average $T_2$ value for the identified peak in the $T_2$ distribution curve of the standardised sample and similarly calculating $T_2$(matrix) which is defined as the weighted average $T_2$ value for the identified peak in the $T_2$ distribution curve of the matrix reference sample;
l. calculating the values of $R_2$(sample) and $R_2$(matrix), where:

$$R_2(\text{sample}) = 1 / T_2(\text{sample}),$$

and

$$R_2(\text{matrix}) = 1 / T_2(\text{matrix});$$

m. calculating the fibre defibrillation parameter FDP of the defibrillated primary cell wall material as

$$FDP = R_2(\text{sample}) - R_2(\text{matrix}).$$

The CPMG $T_2$ relaxation pulse sequence is well-known in the field of NMR spectroscopy (See Effects of diffusion on free precession in nuclear magnetic resonance experiments, Carr, H.Y., Purcell, E.M., Physical Review, Volume 94, Issue 3, 1954, Pages 630-638 / Modified spin-echo method for measuring nuclear relaxation times, Meiboom, S., Gill, D., Review of Scientific Instruments, Volume 29, Issue 8, 1958, Pages 688-691) Suitable time domain NMR spectrometers are well-known to perform this type of spectroscopy are well-known. Similarly, the usual measures to ensure the recording of reliable data are well-known in the field of time domain NMR spectroscopy. For example, the field should be sufficiently homogeneous at the locus where the sample volumes are placed. The field homogeneity can be checked by verifying whether a reference sample of pure water, yields a $T_2$* (T-two-star) for water protons of more than 2 milliseconds.

The inverse Laplace transformation of step i may suitably be carried out using a non-negative least square constraints algorithm Isqnonneg (Lawson, C.L. and R.J. Hanson, Solving Least Squares Problems, Prentice-Hall, 1974, Chapter 23, p. 161), with the regularisation parameter lambda set to 0.2. Software packages suitable for implementing the algorithm and carrying out the transform are well-known, Matlab being an example of such software.

In step j the peak that is selected in the $T_2$ distribution curve of the standardised sample, typically is the dominant peak, if the system is sufficiently homogeneous. In general, the peak that should be selected in the $T_2$ distribution curve is that corresponding to water protons of which the $T_2$ is averaged by diffusion and chemical exchange between bulk and surface sites of the defibrillated primary cell wall material. This peak is particularly well-defined if the defibrillated primary cell wall material is evenly distributed over the standardised sample. In most typical cases, there will be only one such peak, as can be seen in the examples in the Examples section below.

The weighted average $T_2$ in step l is for example suitably calculated by the summation

$$\frac{\sum I(T_2) \cdot T_2}{\sum I(T_2)}$$

Here, $I(T_2)$ is the intensity at value $T_2$ and both summations are over the width of the peak.

A preferred way of establishing the FDP for the cleansing composition is by following the protocol in the way described in the Examples section below for the FDP. The above protocol and the Examples provide methods of measuring the FDP. However, the FDP may also be determined by a different protocol, as long as that protocol would lead to the same physical result, i.e. it would yield the same FDP for a particular cleansing composition as the above protocol.

Combination of parameters

Cleansing compositions wherein the above-specified requirements for the CHP, FHP, and FDP are simultaneously satisfied for more than one of the CHP, FHP, and FDP are also contemplated. For example, a cleansing composition wherein the composition homogeneity parameter CHP has a value as specified hereinabove and simultaneously a fibre defibrillation parameter FDP as defined hereinabove is preferred. Likewise, a cleansing composition wherein the fibre homogeneity parameter FHP has a value as specified hereinabove and simultaneously a fibre defibrillation parameter FDP as defined hereinabove is also preferred.

Methods

Disclosed are methods for preparing a cleansing composition as defined hereinabove. A cleansing composition made according to the present methods surprisingly provides enhanced foam stability, in particular if the composition is diluted to form suds or a lather. These surprising properties are believed to be due to the particular processing conditions and their effect on the primary cell wall material comprising microfibrils.

The methods are methods wherein the cleansing composition comprises water, one or more detergent surfactants, and defibrillated primary cell wall material comprising microfibrils.

The method is preferably a method for preparing a cleansing composition according to the invention as described hereinabove. Thus, any preferences regarding the cleansing composition according to the invention apply here too. The method preferably is a method for preparing a cleansing composition in a form suitable for domestic use (including for example hand dish wash formulations). In particular it is preferred that it is a method for preparing a cleansing composition according to the first aspect of the invention, or according to the second aspect of the invention, or according to the third aspect of the invention.

The primary cell wall material is preferably sourced as indicated for the cleansing composition above. It is particularly preferred that the primary cell wall material includes citrus fibre.

Method according to the first described method

Step ii of the first described method involves dispersing the primary cell wall material in an aqueous phase. Any method to disperse the primary cell wall material is considered, as long as it yields a dispersion that is suitable for the treatment in step iii. Thus, the dispersion step may involve stirring, mixing, or another treatment of relatively low shear, such as treatment with an overhead or inline Silverson mixer.

The aqueous dispersion of step ii comprises between 0.1 and 4.0% by wt. of the primary cell wall material. Preferably, it comprises between 0.1 and 2.5% by wt., more preferably between 0.5 and 2.0% by wt. of the primary cell wall material.

The treatment of step iii to obtain a dispersion comprising defibrillated primary cell wall material involves subjecting the primary cell wall material to mechanical shearing and/or cavitation. To this effect, the treatment includes a high shear treatment step selected from high pressure homogenisation at a pressure of between 500 and 2000 bar or microfluidising at a pressure of between 500 and 2000 bar.
Both high pressure homogenisation and microfluidisation are well-known techniques, involving well-known equipment. Preferably, the high shear treatment step is high pressure homogenisation as specified, more preferably, it is high pressure homogenisation at a pressure of between 500 and 1000 bar, and even more preferably at a pressure of between 600 and 800 bar.

Thus, it is especially preferred that the aqueous phase of step ii comprises between 0.2 and 1.5 wt % of the primary cell wall material and the high shear treatment step of step iii is high pressure homogenisation at a pressure of between 600 and 800 bar.

The precise pressure and the number of passes and/or stages of the treatment - be it high pressure homogenisation or microfluidisation - that is required to obtain the benefits of the present invention may depend for instance on the concentration of the primary cell wall material present and on its level of comminution/pre-treatment before this step, but is easily determined by experimentation.

The treatment in step iii is such that upon this treatment the fibre homogeneity parameter FHP of the defibrillated primary cell wall material is at least 0.022. Here the fibre defibrillation parameter FHP is defined and determined as described above. The defibrillated primary cell wall material preferably has a fibre homogeneity parameter FHP of at least 0.025, more preferably at least 0.030, even more preferably at least 0.035, still more preferably at least 0.040, yet more preferably at least 0.045 and still more preferably at least 0.050. The defibrillated primary cell wall material preferably has a fibre defibrillation parameter FHP of at most 0.20, more preferably at most 0.15 and even more preferably at most 0.10.

Similarly, it is also preferred that the treatment in step iii is preferably such that upon this treatment the fibre defibrillation parameter FDP of the defibrillated primary cell wall material is at least 0.10 Hz. Here the fibre defibrillation parameter FDP is defined and determined as described above. The defibrillated primary cell wall material preferably has a fibre defibrillation parameter FDP of at least 0.11 Hz, more preferably at least 0.12 Hz, even more preferably at least 0.13 Hz, even more preferably at least 0.15 Hz and still more preferably at least 0.18 Hz. The defibrillated primary cell wall material preferably has a fibre defibrillation parameter FDP of at most 0.50 Hz, more preferably at most 0.40 Hz, even more preferably at most 0.30 Hz and still more preferably at most 0.20 Hz.

The FHP and/or FDP can in particular be conveniently determined if the aqueous dispersion consists substantially of water and primary cell wall material, since in that case, the sample preparation step of the protocols to determine the FDP and/or FHP are relatively straight-forward.

Surprisingly beneficial properties of the cleansing composition made by the present method (in terms of enhanced foam stability whilst maintaining other desirable properties) are obtained when the treatment in step iii is such that the above preferred requirements for the FDP and/or the FHP are met.

Constituents of the cleansing composition other than the primary cell wall material are independently mixed into the aqueous phase before step ii, between steps ii and iii, between steps iii and iv or after step iv. These constituents include the one or more detergent surfactants. The other constituents can be mixed at the stage that is most convenient

and/or efficient depending on the type of constituents and the product format as will be known and appreciated by the skilled person. However, care should be taken that the aqueous dispersion in step iii is suitable for the treatment it is subjected to.

The method may suitably involve other routine steps and equipment that are usual and well-known in the field of manufacture of cleansing compositions, in particular with regard to cleansing compositions for domestic use.

Method according to the second described method

The preferences and considerations relating to the method according to the first described method similarly apply to this method. Thus, for instance, the treatment of step iii typically involves one or more high-shear treatments selected from high pressure homogenisation and microfluidising. For this method any number and order of such treatment steps is contemplated as long as the requirements of the FDP and/or FHP are met for the resulting cleansing composition. Other steps may be present in between such multiple shearing steps, including for example the mixing in of other ingredients.

The treatment of step iii is such that that the fibre defibrillation parameter FDP of the defibrillated primary cell wall material is at least 0.10 Hz or the fibre homogeneity parameter FHP of the defibrillated primary cell wall material is at least 0.022. Preferably, the treatment is such that the fibre defibrillation parameter FDP is at least 0.11 Hz, more preferably at least 0.12 Hz, even more preferably at least 0.13 Hz, even more preferably at least 0.15 Hz and still more preferably at least 0.18 Hz. The fibre defibrillation parameter FDP preferably is at most 0.50 Hz, more preferably at most 0.40 Hz, even more preferably at most 0.30 Hz and still more preferably at most 0.20 Hz.

The defibrillated primary cell wall material preferably has a fibre homogeneity parameter FHP of at least 0.025, more preferably at least 0.030, even more preferably at least 0.035, still more preferably at least 0.040, yet more preferably at least 0.045 and still more preferably at least 0.050. The defibrillated primary cell wall material preferably has a fibre defibrillation parameter FHP of at most 0.20, more preferably at most 0.15 and even more preferably at most 0.10.

Cleansing composition obtainable by the methods.

The present invention also relates to a cleansing composition obtainable by a method disclosed, as the method yields cleansing compositions exhibiting desirable properties, including enhanced foam stability by virtue of the particular structure that results from this method.

It is preferred that the cleansing composition is obtainable by the method according to the first described method wherein the aqueous dispersion of step ii comprises between 0.1 and 2.5 wt % of the primary cell wall material and the high shear treatment step of step iii is high pressure homogenisation at a pressure of between 700 and 1000 bar.

Likewise, it is preferred that the cleansing composition is obtainable by the method according to the first and second described method, wherein the treatment in step iii is such that upon this treatment the fibre defibrillation parameter FDP of the defibrillated primary cell wall material is at least 0.10 Hz. Here the fibre defibrillation parameter FDP is defined and determined as described above. The defibrillated primary cell wall material preferably has a fibre defibrillation parameter FDP of at least 0.11 Hz, more preferably at least 0.12 Hz, even more preferably at least 0.13 Hz, even more preferably at least 0.15 Hz and still more preferably at least 0.18 Hz. The defibrillated primary cell wall material preferably has a fibre defibrillation parameter FDP of at most 0.50 Hz, more preferably at most 0.40 Hz, even more preferably at most 0.30 Hz and still more preferably at most 0.20 Hz.

Analogously, it is preferred that cleansing composition is obtainable by either described method, wherein the treatment in step iii is such that upon this treatment the fibre homogeneity parameter FHP of the defibrillated primary cell wall material is at least 0.022. The defibrillated primary cell wall material preferably has a fibre homogeneity parameter FHP of at least 0.025, more preferably at least 0.030, even more preferably at least 0.035, still more preferably at least 0.040, yet more preferably at least 0.045 and still more preferably at least 0.050. The defibrillated primary cell wall material preferably has a fibre defibrillation parameter FHP of at most 0.20, more preferably at most 0.15 and even more preferably at most 0.10.

## Examples

Examples 1 to 3: Skin cleansing composition

Compositions according to the invention were prepared and compared with a comparative example without citrus fibres (see compositions in Table 1)

Preparation of comparative example A

To 702.7 grams of demineralized water in a beaker, 214.3 grams of sodium lauroamphoacetate were added and stirred with an overhead mixer at 100 rpm until a uniform sample was obtained. Then the beaker was placed in a 70°C water bath and 60.0 grams of sodium cocoyl glutamate was added and stirred with an overhead mixer at 100 rpm until uniform. Under continued stirring 18.0 grams of citric acid was slowly added until pH 5.0 was reached. The beaker was then taken from the water bath and 5.0 grams of sodium benzoate was added and stirred until fully dissolved.

Preparation of Examples 1, 2, 3

To 1395.4 grams of demineralized water in a beaker, citrus fibre (Herbacel AQ+type N ex Herbafoods) was added in an amount corresponding to 0.5 wt. % of the final composition. The citrus fibre was allowed to hydrate under stirring with magnet bar for 20 minutes, followed by stirring using the Silverson L4RT-A overhead mixer (screen with 1 mm holes) at 2500 for 20 minutes. Then 428.6 grams of sodium lauroamphoacetate was added and stirred with an overhead mixer at 100 rpm until a uniform sample was obtained. Then the beaker was placed in a 70°C water bath and 120.0 grams of sodium cocoyl glutamate was added and stirred with an overhead mixer at 100 rpm until uniform. Under continued stirring 36.0 grams of citric acid was slowly added until pH 5.0 is reached. The beaker was then taken from the water bath and 10.0 grams of sodium benzoate was added and stirred until fully dissolved. The resulting formulation was homogenized by passing it over high pressure homogenizer (Niro Soavi, Panda NS 1001L) at a pressure of 200 bar (Example 1), 500 bar (Example 2), and 800 bar (Example 3), respectively.

Table 1: compositions of examples 1-3 and comparative example A

| Ingredients | Comparative Example A (wt. %) | Examples 1-3 (wt. %) |
|---|---|---|
| Demineralized water | 70.27 | 69.77 |
| Herbacel AQ+type N (citrus fibres) | -- | 0.50 |
| Sodium lauroamphoacetate (28 wt. % solution in water) | 21.43 | 21.43 |
| Sodium cocoyl Glutamate | 6.00 | 6.00 |
| Citric acid | 1.80 | 1.80 |
| Sodium benzoate | 0.5 | 0.5 |
| Total | 100.0 | 100.0 |

Characterization of the composition determination of the composition homogeneity parameter (CHP)

The composition homogeneity parameter CHP was determined for the cleansing compositions of each of the examples 1-3, and for comparative example A. The protocol to establish the parameter includes three parts: sample preparation, confocal scanning laser microscopy (CSLM), and digital image analysis to calculate the CHP value.

CHP - sample preparation

150 grams of each example was diluted to a citrus fibre concentration of 0.250 wt. % in demineralized water (yielding 300 g of diluted dispersion) using a 500ml plastic beaker of 80mm in diameter. The mixture was stirred using a Silverson L4RT-A overhead mixer (screen with 1 mm holes) at 2000 rpm for 60 seconds. This mixing step ensures that the citrus fibre is evenly distributed over the diluted sample volume. Next, 25 grams of sample was taken from the top layer and transferred into a glass vial.

For each example, a volume of 1 mL of the resulting diluted sample was taken with a Finn pipette (Labsystems 4500, H37095) and deposited in an Eppendorf safelock tube. To this 10 μL of a 0.5 w/v % aqueous solution of Congo Red dye was added with a Finn pipette (Labsystems 4027, H56580). The sample was gently tilted back and forth to

distribute the dye. Care was taken to remove/not introduce air bubbles in the samples, which interfere with imaging. For imaging, a sample holder was filled with the dyed sample material. The sample holder consisted of two cover slides separated by a spacer. The spacer is a rectangular glass slide of 3 mm thick with a circular hole (0.5 cm diameter) in which the sample could be deposited.

CHP - confocal scanning laser microscopy

Confocal scanning laser microscopy (CSLM) was performed on a Leica TCS-SP5 confocal microscope in combination with a DM16000 inverted microscope frame. The diode-Pumped-Solid-State (DPSS) 561 laser emitting at 561 nm was used at a fixed laser power of 58% for imaging with the Congo red dye. For detection, the system was equipped with three PMT (photomultiplier tube) detectors.

Images were taken with a 10x objective with a numerical aperture of 0.40 (section thickness 6.23 μm). Tile scans of 2 by 2 images at, at least, 7 different depths were recorded to yield 25 non-overlapping images for analysis. Care was taken not to image the edges of the sample holder, images were taken at a few micrometers distance from the edge. When samples contain air bubbles care was taken to only record images that do not contain any bubbles in the field of view. The PMTs were adjusted by using the "smart gain" and "smart offset" options to prevent over-saturation of the images. Intensity and gain are then adjusted such that between 0.1 and 5% of the pixels are saturated. CHP values and limits were obtained at an intensity of 58% and a gain of 700 V. The resolution of the images was set to 1024 by 1024 pixels and a line averaging of 3 is used. Each pixel represents a sample area of 1515.2 by 1515.2 nm. After imaging, the individual pictures that make up the tile scan are exported as tiff files with a color depth of 24 bit RGB without incorporating any scale bar (the reconstructured larger tile images are not used in the image analysis).

CHP - digital image analysis

For the image analysis the program ImageJ (freeware downloadable from: http://rsbweb.nlh.gov//ij/) was used together with Microsoft Excel. Each image was converted to an 8 bit grey scale before analysis. In the analysis, images were first normalized (i.e., a histogram stretch) using the "enhanced contrast" option of ImageJ, allowing 0.4% of the pixels to become saturated. After this procedure, the histogram containing the distribution of pixel intensities is calculated. The resulting list containing the number of pixels per channel, in which each channel represents one of the 256 grey scale values in the image was transferred to Microsoft Excel. Before determination of the maximum of the distribution, spikes/outliers were removed from the obtained histogram by visual inspection, considering that a channel displaying a spike has a considerably larger value than the channels immediately adjacent to it (~2 times or higher). When the histogram displays a smooth distribution, the value of the spike is larger than the maximum of this distribution and located on the right or left of the true maximum. After removal, the maximum of the distribution was determined and divided by two. The full width at half maximum (FWHM) was determined by counting the channels that have a value higher or equal to half the maximum. Any channel containing a zero value that is adjacent to a channel with a count higher than half the maximum is included in the count. The obtained channel count was divided by 256 to yield a FWHM number between 0 and 1 for each individual image. The composition homogeneity parameter was then calculated as the arithmetic average of the FWHM values obtained for the individual images of a particular sample. The reported error is the standard deviation of this average. The characterization of the examples in terms of their CHP is summarized in Table 2. The results in Table 2 show that the cleansing formulation with MFC with higher degree of defibrillation have higher CHP.

Table 2: Composition homogeneity parameter CHP

| Example | CHP | Standard deviation |
|---|---|---|
| A | ND | ND |
| 1 | 0.0455 | 0.009 |
| 2 | 0.0780 | 0.010 |
| 3 | 0.0967 | 0.010 |
| ND: not detectable as comparative example A contains no citrus fibres.<br>As seen, CHP was 0.030 and greater and, in the absence of fibers, no CHP is detected. | | |

Foam volume

The formulations of compositions A and 1-3 were aerated using a Kenwood Chef Classic orbital mixer with a

whisk utensil. Demi-water (190 grams) and 10 grams of composition were placed in the bowl of the mixer, followed by mixing for 1 minute at preset 5. The contents of the bowl were transferred to a 1 litre polypropylene beaker (bottom diameter 96 mm, height 182 mm, *ex* Vitlab). In the beaker a liquid layer and a clearly discernible foam layer were formed within several minutes. The volume of the foam was recorded at the start of the experiment, after 30 minutes, and 60 minutes. The resulting foam volumes are presented in Table 3 below. Examples 1 to 3 all had statistically significant more foam volume compared to comparative example A for each recorded incubation time ($r \leq 0.05$ according to Student's T-test with two tailed distribution and two sample equal variance).

Table 3: Foam volume in time of examples 1-3 versus comparative example A

| Incubation time (min) | Foam value (ml ± SD, n=5) | | | |
|---|---|---|---|---|
| | A | 1 | 2 | 3 |
| 0 | 866 ± 43 | 1002 ± 18 | 1028 ± 39 | 1050 ± 19 |
| 30 | 832 ± 47 | 980 ± 22 | 994 ± 40 | 1004 ± 32 |
| 60 | 830 ± 43 | 890 ± 23 | 920 ± 19 | 934 ± 26 |

Table 4 below provides a comparison of the change in foam volume relative to that of the comparative A, according to the formula:

$$R_n(t) = \frac{V_n(t)}{V_A(t)}$$

Here, $R_n(t)$ is the relative foam volume of Example *n (n* being A, 1, 2, or 3) at time *t,* $V_n(t)$ is the foam volume of Example *n* at time *t,* and *VA(t)* is the foam volume of Example A at time *t*.

Table 4: Relative foam volume, relative to comparative example A

| Incubation time (min) | Relative foam value | | | |
|---|---|---|---|---|
| | A | 1 | 2 | 3 |
| 0 | 1.00 | 1.16 | 1.19 | 1.21 |
| 30 | 1.00 | 1.18 | 1.19 | 1.21 |
| 60 | 1.00 | 1.07 | 1.11 | 1.13 |

Table 4 shows that at t = 0 minutes the relative foam volume (relative to the volume of the comparative example A) increased for all three Examples (1-3), indicating a foam boosting effect. This boost in relative foam volume over comparative sample A, remained present - in a statistically significant way - *over* at least 60 minutes.

## Claims

**1.** A cleansing composition comprising:

1) 0.5 to 35% by wt. of total composition of a surfactant system which contains:

a) 0.5 to 25% by wt. of total composition anionic surfactant and
b) 0 to 20% by wt. of a co-surfactant selected from the group consisting of non-ionic surfactant, cationic surfactant, amphoteric surfactant and mixtures thereof; where an N-acyl salt of polycarboxylic acid is present at a level of 50% or more of all surfactant (both (a) and (b)); or is present at a level of 50% or more of anionic surfactant (a);

2) 0 to 30% by wt. of a hair or skin benefit agent;
3) 0.05 to 4.0 wt. % of a primary cell wall material comprising microfibers wherein:

a) the primary cell wall material is sourced from plant parenchymal tissue;
b) 80% or more, preferably 80% to 100%, more preferably 85% to 100% of the microfibrils have a diameter of less than 50 nanometers (nm); and

4) balance of water,

wherein the primary cell wall material is defibrillated,
wherein the cleansing composition has a composition homogeneity parameter (CHP) of 0.030 and greater, according to the protocol as defined herein.

**2.** A composition according to claim 1, wherein the cell wall material has a fiber homogeneity parameter (FHP) of 0.030 and greater, according to the protocol as defined herein.

**3.** A composition according to any of claims 1 or 2, wherein the cell wall has a fiber defibrillation parameter (FDP) of 0.10 Hz or greater, according to the protocol as defined herein.

**4.** A composition according to any of claims 1 to 3, wherein the salt of polycarboxylic acid is a salt of dicarboxylic acid.

**5.** A composition according to claim 4, wherein the salt of dicarboxylic acid is a salt of glutamate.

**6.** A composition according to any of claims 1 to 5, wherein pH of the composition is 3 to 6.5.

**Patentansprüche**

**1.** Reinigungszusammensetzung, umfassend:

1) 0,5 bis 35 Gew.-% der Gesamtzusammensetzung von einem Tensidsystem, das enthält:

a) 0,5 bis 25 Gew.-% der Gesamtzusammensetzung anionisches Tensid und
b) 0 bis 20 Gew.-% eines Cotensids, ausgewählt aus der Gruppe bestehend aus nichtionischem Tensid, kationischem Tensid, amphoterem Tensid und Mischungen davon; wobei ein N-Acylsalz von Polycarbonsäure in einer Menge von 50% oder mehr des gesamten Tensids (sowohl (a) als auch (b)) vorhanden ist; oder in einer Menge von 50% oder mehr von anionischem Tensid (a) vorhanden ist;

2) 0 bis 30 Gew.-% eines Haar- oder Hautpflegemittels;
3) 0,05 bis 4,0 Gew.-% eines primären Zellwandmaterials, umfassend Mikrofasern, wobei:

a) das primäre Zellwandmaterial aus pflanzlichem Parenchymgewebe stammt;
b) 80% oder mehr, bevorzugt 80% bis 100%, bevorzugter 85% bis 100%, der Mikrofibrillen haben einen Durchmesser von weniger als 50 Nanometern (nm) aufweisen; und

4) Rest Wasser,

wobei das primäre Zellwandmaterial defribilliert ist,
wobei die Reinigungszusammensetzung einen Zusammensetzungs-Homogenitätsparameter (CHP) von 0,030 und mehr aufweist, gemäß dem Protokoll wie hierin definiert.

**2.** Zusammensetzung nach Anspruch 1, wobei das Zellwandmaterial einen Faserhomogenitätsparameter (FHP) von 0,030 und mehr aufweist, gemäß dem Protokoll wie hierin definiert.

**3.** Zusammensetzung nach irgendeinem der Ansprüche 1 oder 2, wobei die Zellwand einen Faserdefibrillationsparameter (FDP) von 0,10 Hz oder mehr aufweist, gemäß dem Protokoll wie hierin definiert.

**4.** Zusammensetzung nach irgendeinem der Ansprüche 1 bis 3, wobei das Salz von Polycarbonsäure ein Salz von Dicarbonsäure ist.

**5.** Zusammensetzung nach Anspruch 4, wobei das Salz von Dicarbonsäure ein Salz von Glutamat ist.

6. Zusammensetzung nach irgendeinem der Ansprüche 1 bis 5, wobei der pH der Zusammensetzung 3 bis 6,5 beträgt.

## Revendications

1. Composition de nettoyage comprenant :

    1) 0,5 à 35 % en masse de composition totale d'un système de tensioactif qui contient :

        a) 0,5 à 25 % en masse de composition totale de tensioactif anionique et
        b) 0 à 20 % en masse d'un co-tensioactif choisi dans le groupe consistant en tensioactif non-ionique, tensioactif cationique, tensioactif amphotère et des mélanges de ceux-ci ; où un sel de N-acyle d'acide polycarboxylique est présent à une teneur de 50 % ou plus de tous les tensioactifs (à la fois (a) et (b)) ; ou est présent à une teneur de 50 % ou plus de tensioactif anionique (a) ;

    2) 0 à 30 % en masse d'agent bénéfique pour les cheveux ou la peau ;
    3) 0,05 à 4,0 % en masse d'un matériau de paroi de cellule primaire comprenant des microfibres dans laquelle :

        a) le matériau de paroi de cellule primaire provient de tissu parenchymateux de plante ;
        b) 80 % ou plus, de préférence de 80 % à 100 %, encore mieux de 85 % à 100 % des microfibrilles présentent un diamètre inférieur à 50 nanomètres (nm) ; et

    4) le reste d'eau,

    dans laquelle le matériau de paroi de cellule primaire est défibrillé,
    dans laquelle la composition de nettoyage présente un paramètre d'homogénéité de composition (CHP) de 0,030 et supérieur, selon le protocole comme défini ici.

2. Composition selon la revendication 1, dans laquelle le matériau de paroi de cellule présente un paramètre d'homogénéité de fibre (FHP) de 0,030 et supérieur, selon le protocole comme défini ici.

3. Composition selon l'une quelconque des revendications 1 ou 2, dans laquelle la paroi de cellule présente un paramètre de défibrillation de fibre (FDP) de 0,10 Hz ou supérieur, selon le protocole comme défini ici.

4. Composition selon l'une quelconque des revendications 1 à 3, dans laquelle le sel d'acide polycarboxylique est un sel d'acide dicarboxylique.

5. Composition selon la revendication 4, dans laquelle le sel d'acide dicarboxylique est un sel de glutamate.

6. Composition selon l'une quelconque des revendications 1 à 5, dans laquelle le pH de la composition est de 3 à 6,5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2015076478 W, Unilever
- EP 2015081008 W
- US 20150159120 A, Fernandez-Prieto
- EP 2603196 A


### Non-patent literature cited in the description


- **CARR, H.Y. ; PURCELL, E.M.** Effects of diffusion on free precession in nuclear magnetic resonance experiments. *Physical Review,* 1954, vol. 94 (3), 630-638
- **MEIBOOM, S. ; GILL, D.** Modified spin-echo method for measuring nuclear relaxation times. *Review of Scientific Instruments,* 1958, vol. 29 (8), 688-691
- **LAWSON, C.L. ; R.J. HANSON.** Solving Least Squares Problems. Prentice-Hall, 1974, 161